# EUROPÄISCHE PATENTSCHRIFT

(11) **EP 1 325 504 B1**
(45) Veröffentlichungstag und Bekanntmachung des Hinweises auf die Patenterteilung: **15.06.2005**
(21) Anmeldenummer: 01980490.5
(22) Anmeldetag: 12.10.2001
(51) Int. Cl.: H01F 41/06, H01F 5/00, H01F 5/06, A61N 1/40

(54) **SPULE FÜR MAGNETFELDTHERAPIE**
COIL FOR MAGNETIC FIELD THERAPY
BOBINE POUR THERAPIE PAR CHAMP MAGNETIQUE

(30) Priorität: 13.10.2000 DE 10050760
(43) Veröffentlichungstag der Anmeldung: 09.07.2003
(73) Patentinhaber: Elmag GmbH, 81673 München (DE)
(72) Erfinder: HOLZER, Josef, 81675 München (DE)
(74) Vertreter: Klunker . Schmitt-Nilson . Hirsch
(86) Internationale Anmeldenummer: PCT/EP2001/011851
(87) Internationale Veröffentlichungsnummer: WO 2002/031845

(56) Entgegenhaltungen:
- EP-A- 0 136 265
- WO-A-94/28969
- WO-A-95/27533

## Beschreibung

Die Erfindung betrifft flexible Spulen aus flexiblem isoliertem Draht für die Magnetfeldtherapie.

### Nächstliegender Stand der Technik

WO 94/28969 A beschreibt die Verwendung einer flexiblen Spule aus einem einadrigen oder mehradrigen Draht für die Magnetfeldtherapie.

Bei der Magnetfeldtherapie werden stromdurchflossene Spulen zur Erzeugung der für die Therapie erforderlichen Magnetfelder verwendet. Dafür werden typischerweise aus einfachem isoliertem Kupferkabel gewickelte Ringspulen verwendet. Derartige Ringspulen bestehen aus einem Kabelbündel, das einen in etwa kreisförmigen Querschnitt besitzt, d.h. sie haben eine torusförmige Gestalt. Das Kabelbündel ist dabei z.B. von einem Klebeband in der Torusform gehalten. Das verwendete Kupferkabel hat typischerweise wenige, beispielsweise bis zu 20 Adern und besitzt deshalb eine gewisse Eigenstabilität, die erwünscht ist, um die Ringform der Spule beizubehalten. Diese Spulen haben häufig eine Hülle aus Textilmaterial und werden zur Behandlung auf den Körper der zu behandelnden Person oder des zu behandelnden Tieres aufgelegt und können daran beispielsweise mit Klettverschlüssen und/oder Gurten befestigt werden. Dazu ist es günstig, wenn die Spule flexibel ist. Das erlaubt einerseits das In-Anlage-Bringen der Spule mit dem zu behandelnden Körperteil und stellt andererseits die Bewegungsfähigkeit des behandelten Menschen während der Behandlung sicher.

Die Magnetfeldtherapie und damit die Verwendung der hier beschriebenen Spule eignet sich gleichermaßen für die Behandlung von Menschen und Tieren. Wenn im Nachfolgenden lediglich auf die Behandlung des Menschen eingegangen wird, soll das keinesfalls die Behandlung von Tieren ausschließen. Vielmehr ist die Erfindung grundsätzlich in gleicher Weise für die Behandlung von Tieren wie von Menschen geeignet.

Die erforderliche Flexibilität ist bei den konventionellen Spulen nicht in ausreichendem Maße gegeben.

Es ist deshalb die Aufgabe der vorliegenden Erfindung eine Spule bereitzustellen, die einerseits flexibler ist als die bislang verwendeten Spulen und die andererseits ihre Grundform trotz.dieser Flexibilität beibehält.

Erfindungsgemäß wird diese Aufgabe gelöst von einer flexiblen Spule gemäß Anspruch 1.

Die Flächigkeit oder 2-Dimensionalität hat den Vorteil, daß die Flexibilität der flachen Spule in der Ebene der Fläche trotz des gleichen Drahtmaterials wie bei den Spulen des Stands der Technik verglichen mit den bisher verwendeten torusförmigen Spulen relativ groß ist. Die Flexiblität der Spule ist vergleichbar mit der Flexibilität eines Blatt Papiers.

Das flächige Gebilde kann beispielsweise eine Scheibenform oder eine Zylinderform oder jede andere Form besitzen. Scheibenförmige Spulen eignen sich beispielsweise zum Auflegen auf einen Behandlungsbereich, der im wesentlichen rechtwinklig vom Magnetfeld durchsetzt werden soll, während eine zylinderförmige Spule beispielsweise über einen Arm oder ein Bein geschoben werden kann, um diesen Bereich in Längsrichtung mit dem Magnetfeld zu durchsetzen. Die flächige Spule ist bei solchen Anwendungen besonders bevorzugt, wo wie beispielsweise bei Behandlungen der Wirbelsäule sich der Patient auf die Spule legt. Die Spulen aus dem Stand der Technik hatten gerade bei derartigen Anwendungen eklatante Nachteile wegen ihrer Dicke und Steifigkeit. In manchen Fällen bestand sogar die Gefahr, daß sich die Beschwerden des Patienten wegen ungünstiger Haltung verursacht durch die Dicke der Spule verschlimmerten.

Durch die Verbindung der einzelnen Isolierungsbereiche miteinander erhält die Spule eine gewisse Eigenstabilität, die die relative Anordnung der Isolierungsbereiche zueinander sicherstellt und diese im wesentlichen beibehalten kann. Da die Spule zur Verwendung typischerweise von einer Hülle umgeben ist, und an dieser auch befestigt ist, reicht es im Prinzip aus, wenn die Spule diese Eigenstabilität für die Dauer der Herstellung beibehält. Eine dauernde Verbindung zwischen den einzelnen Isolierungsbereichen ist jedoch bevorzugt. Es ist auch bevorzugt, wenn die Isolierungsbereiche über die gesamte Länge miteinander verbunden sind.

Der Draht der Spule ist eine Schaltlitze mit mindestens 50, 100, 200 oder gar 250 und mehr dünnen Adern. Litzen besitzen generell eine umso größere Flexibilität, je dünner die einzelnen Adern in der Litze sind. Solche Schaltlitzen haben gegenüber den bisher verwendeten Drähten eine wesentlich größere Flexibilität, was besonders bevorzugt ist. Schaltlitzen sind problemlos und -relativ günstig in der erforderlichen Stärke käuflich zu erwerben. Mangels Eigenstabilität konnten diese bisher für den genannten Zweck nicht verwendet werden.

Vorzugsweise sind die einander berührenden Bereiche der Isolierung miteinander verschweißt. Ein Verschweißen kann durch Wärmeeinwirkung relativ einfach und mit wenig Zeitaufwand realisiert werden, indem bei der Herstellung ein relativ großer Strom durch die Spule geschickt wird oder die Spule mit Infrarotstrahlung erwärmt wird. Die damit verbundene Wärmeentwicklung schmilzt die Isolierung des Drahtes an, so daß sich die einander berührenden Isolationsbreiche miteinander verbinden können. Andere Verbindungsmöglichkeiten, z.B. Kleben, können auch vorgesehen sein. Das Verschweißen hat neben den genannten Vorteilen bei der Herstellung den Vorteil, daß kein zusätzliches Material aufgebracht werden muß. Außerdem kann eine Schweißverbindung besonders dauerhaft sein, da keine Materialpaarung mit Materialien unterschiedlicher Materialeigenschaften vorliegt.

Vorzugsweise ist die Spule flach und der Draht spiralförmig gewickelt, so daß die radial nach innen zum Mittelpunkt der Spule hin gerichteten Bereiche der Isolierung des Drahts mit den radial nach außen gerichteten Bereichen des Drahts in Anlage sind. Eine derartige flache Spule läßt sich durch Wickeln um einen runden oder eckigen Spulenkern relativ einfach und kostengünstig herstellen.

Vorzugsweise weist die Spule eine Lage aus spiralförmig gewickeltem Draht auf. Die Spule kann aber auch mehrere Lagen aus spiralförmig gewickeltem Draht aufweisen. Einlagige Spulen sind wegen deren größerer Flexiblität bevorzugt.

Vorzugsweise weist die Spule eine Hülle aus einem hautfreundlichen, flexiblen Material, insbesondere Textilmaterial, auf. Diese Hülle dient einerseits als ein Schutz für die Spule und andererseits dem Komfort des Benutzers. Außerdem können an dem Hüllenmaterial besonders einfach Befestigungsmittel angebracht sein.

Vorzugsweise weist die Spule ein elastisch nachgiebiges Material auf. Dafür eignet sich beispielsweise Schaumstoffmaterial oder ähnliche Materialien. Das erhöht ebenfalls den Komfort für den Benutzer und dient dem Schutz der Spule. Letzteres ist insbesondere dann der Fall, wenn das Material nicht nur auf die Spule gelegt ist, sondern mit dieser z.B. durch Kleben oder Schweißen verbunden ist. Deshalb ist der Draht der Spule vorzugsweise zwischen zwei Lagen aus elastisch nachgiebigem Material vorgesehen und mit diesem beispielsweise durch Kleben verbunden.

Die flexibler Spulen aus flexiblem isoliertem Draht kann nach einem Verfahren, aufweisend die folgenden Schritte, hergestellt werden:
(a) Bereitstellen eines Wickelkerns;
(b) Bewickeln des Wickelkerns mit dem Draht derart, daß einzelne Bereiche der Isolierung miteinander in Anlage sind und so ein flächiges Gebilde bilden;
(c) Verbinden der Berührungsstellen miteinander; und
(d) Entfernen der Spule von dem Wickelkern.

Wie bereits ausgeführt wurde, ist die Herstellung der Spule über einem Wickelkern besonders einfach und entsprechend kostengünstig.

Vorzugsweise erfolgt das Verbinden der Isolierungen an den Berührungsstellen miteinander mittels Wärme. Alternativ kann auf die um den Wickelkern gewickelte Spule auch Klebstoff aufgebracht werden, um sie zu verkleben. Geeignetes Material der Isolierung vorausgesetzt, kann das Isolierungsmaterial auch durch ein Lösungsmittel, z.B. Aceton, angelöst werden. Die erweichten, einander berührenden Bereiche der Isolierung verbinden sich miteinander, sobald das Lösungsmittel sich verflüchtigt. Auch andere Isolierungsmaterialien, die anders als durch Wärme oder Lösungsmittel (beispielsweise durch Infrarotstrahlung) aktivierbar sind, können verwendet werden.

Vorzugsweise wird die Wärme zum Verbinden durch Stromfluß durch die Spule erzeugt. Das erlaubt eine besonders einfache und zeitsparende Herstellung der Spule, was sich wegen der erforderlichen Wickelkerne und ggf. Wickelmaschinen besonders starkauf die Herstellungskosten auswirkt.

Vorzugsweise ist zum Herstellen einer flachen, spiralförmig gewickelten Spule der Wickelkern auf einer ebenen Unterlage angeordnet und der Draht wird spiralförmig um den Wickelkern gewickelt, so daß die radial nach innen zum Mittelpunkt der Spule hin gerichteten Bereiche der Isolierung des Drahts mit den radial nach außen gerichteten Bereichen des Drahts in Anlage sind. Die ebene Unterlage kann dafür horizontal oder vertikal angeordnet sein, wobei eine horizontale Anordnung bevorzugt ist. Die Unterlage dient als eine Anlage oder Führung für den Draht beim Bewickeln des Wickelkerns.

Vorzugsweise werden mehrere Lagen von Draht axial, d.h. in der Richtung der Rotationsachse beim Bewickeln, nebeneinander gewickelt.

Vorzugsweise hat der Wickelkern die Breite der zu wickelnden Spule, und vorzugsweise sind an beiden Seiten des Wickelkerns ebene Begrenzungen vorgesehen, die dem Draht beim Wickeln seitlichen Halt geben. Es soll festgehalten werden, daß der eigentliche Wickelkörper breiter sein kann als die Breite der zu wickelnden Spule. Die Breite des Wickelkerns wird durch den Abstand der Begrenzungen voneinander festgelegt. Mindestens eine der Begrenzungen kann auf dem Wickelkörper in Axialrichtung an unterschiedlichen Stellen vorgesehen werden, um verschiedene Drahtstärken mit der gleichen Herstellungsvorrichtung herstellen zu können und um mehrlagige Spulen herstellen zu können. Der Draht wird beim Bewickeln des Wickelkerns zwischen den beiden Begrenzungen geführt, so daß ein seitliches Ausbrechen beim Bewickeln und beim anschließenden Verbinden der Isolationsbereiche miteinander vermieden ist. Diese Begrenzungen erlauben eine reproduzierbare gleichmäßige Gestalt der fertigen Spule.

Vorzugsweise wird zum Bewickeln des Wickelkerns dieser maschinell angetrieben.

Die Erfindung wird nachfolgend anhand eines zeichnerisch dargestellten Ausführungsbeisepiels noch näher erläutert. Es zeigen:
- Fig. 1: eine flache, spiralförmig um einen Wickelkern gewickelte Spule in Draufsicht;
- Fig. 2: einen Querschnitt durch eine Spule in einer Hülle aus Textilmaterial;
- Fig. 3: eine Spule aus dem Stand der Technik.

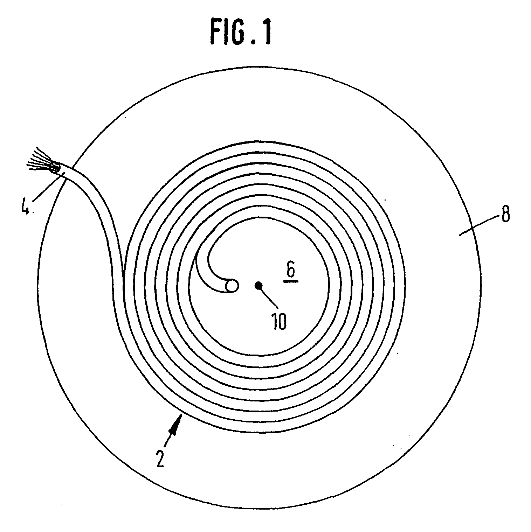

In der Fig. 1 erkennt man eine Spule 2 aus einem Draht 4, der über einen Wickelkern 6 gewickelt ist. Man erkennt ferner eine Unterlage oder Begrenzung 8, die beim Wickeln und anschließenden Verbinden der Isolationsbereiche des Drahts 4 diesem eine Abstützung und Führung liefert. Die Unterlage 8 hat in der Zeichnung im wesentlichen Kreisform. Sie kann aber jede Form besitzen, solange sie als Abstützung für den Draht 4 dienen kann. Eine zweite, nicht gezeigte Begrenzung 8 wird zum Wickeln und Verbinden über dem Draht 4 in etwa eine Drahtstärke von der Unterlage 8 entfernt angeordnet, um dem Draht 4 beidseitig eine Führung zu geben.

Der Draht 4 ist eine isolierte Schaltlitze mit mindestens 50 Adern. Als Leitermaterial ist Kupfer bevorzugt. Wegen ihrer Flexibilität ist eine Schaltlitze mit 250 und mehr Adern bevorzugt. Die Isolation des Drahts ist typischerweise aus Polyvinylchlorid (PVC), das wegen seiner thermoplastischen Eigenschaften günstig ist. Insbesondere kann es durch Erwärmung erweicht werden, wodurch aneinander anliegende Bereiche der Isolierung miteinander verschweißt werden können. Die Isolation kann auch aus anderen Materialien bestehen.

In der Fig. 1 erkennt man weiter, daß von der Rotationsachse 10 weg, radial nach außen gerichtete Bereiche der inneren Wicklungslagen mit radial nach innen gerichteten Bereichen weiter außen liegender Wicklungslagen in Anlage sind. Diese Bereiche werden im Verlauf der Herstellung der Spule 2 miteinander verbunden.

Spulen mit mehr als einer Lage in axialer Richtung können für manche Anwendungen bevorzugt sein. Derartige Spulen 2 können beispielsweise hergestellt werden, indem die erste axiale Lage zwischen den zwei Begrenzungen gewickelt und verbunden wird, wie vorangehend ausgeführt, und danach die "obere" Begrenzung 8 in der Ansicht der Fig. 1 um etwa eine Drahtstärke nach außen versetzt wird, die zweite Drahtlage, beispielsweise von dem inneren Ende der ersten Lage ausgehend, gewickelt wird und erneut ein Isolationsmaterial-Verbindungsschritt erfolgt, bei dem günstigerweise nicht nur die einzelnen Bereiche der zweiten Lage wie bei der ersten Lage miteinander verbunden werden, sondern auch aneinander anliegende Bereiche der ersten axialen Lage und der zweiten axialen Lage miteinander verbunden werden.

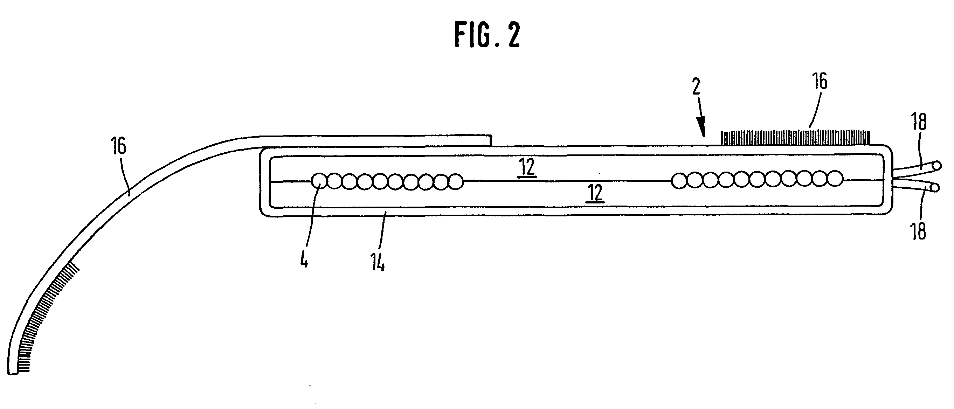

In der Fig.2 erkennt man die Spule 2, die aus dem Draht 4 hergestellt ist. Die entsprechenden Bereiche der Isolierung des Drahts 4 sind miteinander verbunden. Die Spule 2 ist zwischen zwei Lagen aus einem elastisch nachgiebigen Material 12, z.B. Schaumstoff, angeordnet und mit diesem verbunden. Dazu sind die Lagen aus dem elastisch nachgiebigen Material 12 auch an den entsprechenden Berührungsbereichen miteinander verbunden. Üblicher Klebstoff kann beispielsweise dafür verwendet werden.

Die Kombination aus Spule 2 und elastisch nachgiebigem Material 12 ist in einer Hülle 14 aus hautfreundlichem Material, z.B. Textilmaterial oder Leder, etc., angeordnet. Man erkennt, wie die Drahtenden 18 an einer Stelle aus der Hülle 14 herausgeführt sind. An der Hülle 14 können Befestigungselemente 16 zum Anbringen der Spule 2 vorgesehen sein. In der Fig. 2 ist als Befestigungselement 16 ein Band mit Klettverschluß dargestellt.

Die in der Fig. 2 dargestellte Spule 2 ist besonders geeignet, um um einen Glenkbereich, z.B. das Knie oder der Ellenbogen, des Patienten herum angebracht zu werden. Sie ist wegen ihrer Flexibilität bestens daran angepaßt sich an diesen Bereich anzuformen und außerdem dem Patienten auch in diesem Gelenkbereich eine gewisse Beweglichkeit zu geben.

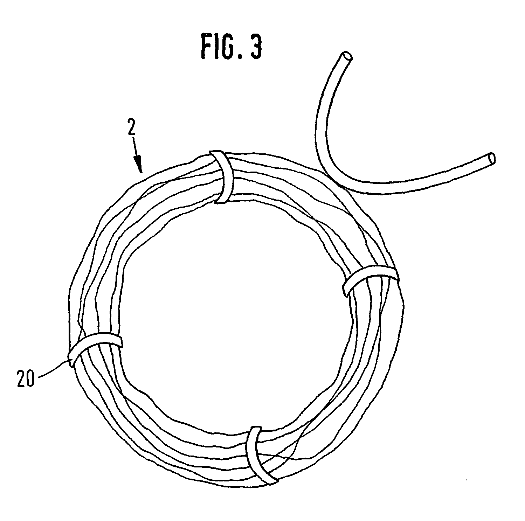

In der Fig. 3 ist eine Spule 2 aus dem Stand der Technik gezeigt. Man erkennt ein torusförmiges Kabelbündel, welches von Bändern 20 mehr schlecht als recht zusammengehalten ist. Durch seine 3-dimensionale Gestalt ist die Flexibilität der Spule 2 deutlich beinträchtigt. Außerdem macht sich die Dicke der Spule 2 für den Patienten besonders bei solchen Anwendungen unangenehm bemerkbar, wo der Patient auf der Spule 2 sitzt oder liegt. Flache mattenartige Spulen, wie sie durch die Erfindung jetzt bereitgestellt werden, gab es bisher nicht.

## Patentansprüche

1. Flexible Spule (2) zur Verwendung in der Magnetfeldtherapie aus einer flexiblen, isolierten Schaltlitze (4), mit mindestens 50 Adern, wobei die Schaltlitze (4) derart gewickelt ist, daß einzelne Bereiche der Isolierung miteinander in Anlage sind und so ein flächiges Gebilde bilden, wobei die einander berührenden Bereiche der Isolierung der Schaltlitze (4) miteinander mindestens bereichsweise verbunden sind.

2. Flexible Spule (2) nach Anspruch 1, **dadurch gekennzeichnet, daß** die einander berührenden Bereiche der Schaltlitze (4) miteinander verschweißt sind.

3. Flexible Spule (2) nach Anspruch 1 oder 2, **dadurch gekennzeichnet, daß** die Spule (2) flach ist und die Schaltlitze (4) spiralförmig gewickelt ist, so daß die radial nach innen zum Mittelpunkt der Spule (2) hin gerichteten Bereiche der Isolierung der Schaltlitze (4) mit den radial nach außen gerichteten Bereichen der Schaltlitze (4) in Anlage sind.

4. Flexible Spule (2) nach Anspruch 3, **dadurch gekennzeichnet, daß** die Spule (2) eine Lage aus spiralförmig gewickelter Schaltlitze (4) aufweist.

5. Flexible Spule (2) nach Anspruch 3, **dadurch gekennzeichnet, daß** die Spule (2) mehrere Lagen aus spiralförmig gewickelter Schaltlitze (4) aufweist.

6. Flexible Spule (2) nach einem der Ansprüche 1 bis 5, **dadurch ge-kennzeichnet, daß** die Spule (2) eine Hülle (14) aus einem hautfreundlichen, flexiblen Material, insbesondere Textilmaterial, aufweist.

7. Flexible Spule (2) nach einem der Ansprüche 1 bis 6, **dadurch gekennzeichnet, daß** die Schaltlitze (4) von einem elastisch nachgiebigen Material (12) eingeschlossen ist.

8. Flexible Spule (2) nach Anspruch 7, **dadurch gekennzeichnet, daß** die Schaltlitze (4) zwischen zwei Lagen aus elastisch nachgiebigen Material (12) vorgesehen ist und damit verbunden ist.

## Claims

1. Flexible coil (2) for use in magnetic field therapy made from a flexible, insulated switch wire (4) with at least 50 strands, whereby the switch wire (4) is wound in such a way that individual sections of the insulation are in contact with one another and so form a two-dimensional structure whereby the sections of the switch wire (4) insulation touching one another are connected to one another, at least in sections.

2. Flexible coil (2) in accordance with claim 1, **characterised in that** the sections of the switch wire (4) touching one another are welded together.

3. Flexible coil (2) in accordance with claim 1 or 2, **characterised in that** the coil (2) is flat and the switch wire (4) is wound spirally so that the sections of the switch wire (4) insulation which run radially inwards towards the central point of the coil (2) are in contact with the sections of the switch wire (4) running radially outwards.

4. Flexible coil (2) in accordance with claim 3, **characterised in that** the coil (2) has a layer made from spirally wound switch wire (4).

5. Flexible coil (2) in accordance with claim 3, **characterised in that** the coil (2) has several layers made from spirally wound switch wire (4).

6. Flexible coil (2) in accordance with any of the claims 1 to 5, **characterised in that** the coil (2) has a casing (14) made from a eudermic, flexible material, in particular a textile material.

7. Flexible coil (2) in accordance with any of the claims 1 to 6, **characterised in that** the switch wire (4) is enclosed by a pliable elastic material (12).

8. Flexible coil (2) in accordance with claim 7, **characterised in that** the switch wire (4) is located between two layers made from a pliable clastic material (12) and is connected to the same.

## Revendications

1. Bobine souple (2) utilisée dans la thérapie par champ magnétique et formée d'une tresse de connexion (4) souple, isolée, avec au moins 50 brins, qui est enroulée d'une manière telle que des zones particulières de l'isolant soient en contact mutuel et forment ainsi un article plat, les zones en contact mutuel de l'isolant de la tresse de connexion (4) étant liées entre elles au moins localement.

2. Bobine souple selon la revendication 1, **caractérisée par le fait que** les zones en contact mutuel de la tresse de connexion (4) sont soudées entre elles.

3. Bobine souple selon la revendication 1 ou 2, **caractérisée par le fait que** la bobine (2) est plate et que la tresse de connexion (4) est enroulée en spirale de manière telle que les zones de l'isolant de la tresse de connexion (4) tournées radialement vers le centre de la bobine (2) soient en contact avec les zones de l'isolant de la tresse de connexion (4) tournées radialement vers l'extérieur.

4. Bobine souple selon la revendication 3, **caractérisée par le fait que** la bobine (2) comporte une couche de tresse de connexion (4) enroulée en spirale.

5. Bobine souple selon la revendication 3, **caractérisée par le fait que** la bobine (2) comporte plusieurs couches de tresse de connexion (4) enroulée en spirale.

6. Bobine souple selon une des revendications 1 à 5, **caractérisée par le fait que** la bobine (2) présente une enveloppe (14) en un matériau souple compatible avec la peau, plus particulièrement en un matériau textile.

7. Bobine souple selon une des revendications 1 à 6, **caractérisée par le fait que** la tresse de connexion (4) est enfermée dans un matériau déformable élastiquement.

8. Bobine souple selon la revendication 7, **caractérisée par le fait que** la tresse de connexion (4) est prévue entre deux couches de matériau (12) déformable élastiquement et est liée à celles-ci
